# EUROPÄISCHE PATENTSCHRIFT

(11) **EP 4 142 833 B1**
(45) Veröffentlichungstag und Bekanntmachung des Hinweises auf die Patenterteilung: **04.03.2026**
(21) Anmeldenummer: 21719570.0
(22) Anmeldetag: 14.04.2021
(51) Int. Cl.: A61M 5/24, A61M 5/315

(54) **ELEKTRONISCHES ZUSATZMODUL FÜR INJEKTIONSGERÄTE**
ELECTRONIC ADD-ON MODULE FOR INJECTION DEVICE
MODULE SUPPLÉMENTAIRE ÉLECTRONIQUE POUR DISPOSITIF D'INJECTION

(30) Priorität: 30.04.2020 CH 5122020
(43) Veröffentlichungstag der Anmeldung: 08.03.2023
(73) Patentinhaber: Ypsomed AG, 3401 Burgdorf (CH)
(72) Erfinder: SCHEURER, Simon, 3006 Bern (CH); KÜHNI, Florian, 3007 Bern (CH)
(74) Vertreter: Kleiner, Stefan
(86) Internationale Anmeldenummer: PCT/EP2021/059609
(87) Internationale Veröffentlichungsnummer: WO 2021/219371

(56) Entgegenhaltungen:
- WO-A1-2018/013419
- WO-A1-2019/001919
- WO-A1-2020/058875
- CH-A2- 709 878
- CH-A2- 713 129
- US-A1- 2020 114 087

## Beschreibung

### TECHNISCHES GEBIET

Die vorliegende Erfindung betrifft das Gebiet der medizinischen Injektionsgeräte zur Verabreichung von flüssigen Substanzen, insbesondere von Medikamenten oder medizinischen Substanzen wie Insulin- und Hormonpräparationen. Die Erfindung bezieht sich auf ein portables elektronisches Zusatzmodul zum Aufsetzen auf ein medizinisches Injektionsgerät und auf ein Injektionsgerät mit integrierter Elektronik.

### HINTERGRUND

Unterschiedliche Infusions- und Injektionsgeräte können Patienten bei einer kontrollierten subkutanen oder intramuskulären Verabreichung einer Dosis eines Medikamentes aus einem Reservoir unterstützen. Während bei Infusionssystemen eine Kanüle während längerer Zeit im Körper des Patienten verbleibt werden Injektionsgeräte nach jeder Abgabe wieder von der Injektionsstelle entfernt. In Einweg-Injektionsgeräten ist das Reservoir, beispielsweise eine Fertigspritze, nicht dazu vorgesehen, durch den Patienten ersetzt oder wiederaufgefüllt zu werden. In Mehrweg-Injektionsgeräten dagegen kann das Reservoir, beispielsweise eine Karpule, durch den Patienten wieder aufgefüllt oder ersetzt werden. Ein automatisches Injektionsgerät verfügt über einen Motor oder eine gespannte Feder als Energiequelle zum Antrieb einer Kolbenstange und zur Bewegung eines Kolbens im Reservoir, während bei einer Ausschüttung mit einem manuellen Injektionsgerät die Kolbenstange unmittelbar durch eine Kraft des Patienten bewegt wird.

Diabetes wird oft durch Abgabe von Insulin mittels eines Injektionsgerätes in Form eines Insulin-Pens mit einstellbarer Dosis behandelt. Der Pen verfügt über ein längliches Gehäuse mit einem distalen Ende zur Aufnahme einer Kanüle oder Hohlnadel und mit einem der Injektionsstelle abgewandten proximalen Ende. Ein Insulin-Pen kann als Ein- oder Mehrweg-Pen, automatisch oder manuell betrieben werden. Die abzugebende Insulindosis wird normalerweise eingestellt durch Drehen eines Dosierknopfs und gleichzeitiger Kontrolle einer Dosisanzeige des Insulin-Pens. Um eine Insulinabgabe in Echtzeit zu überwachen, beispielsweise zur Verhinderung von Fehlmanipulationen, oder zur Nachverfolgung von mehreren Abgaben über einen längeren Zeitraum, können Informationen betreffend Insulin-Typ, Dosisgrösse, und Zeitpunkt sowie weiterer Umstände jeder Dosisabgabe gesammelt werden.

Die Patentanmeldung EP 3572107 A1 zeigt zu diesem Zweck ein elektronisches Zusatzmodul, welches seitlich auf ein Injektionsgerät aufgesetzt und daran befestigt oder geklemmt werden kann. Das Zusatzmodul umfasst einen Energiespeicher und einen Sensor zur Detektion eines Zustandes des Injektionsgerätes und/oder zur Überwachung eines laufenden Ausschüttvorganges. Ein mehrfach verwendbares Zusatzmodul dieser Art bietet sich insbesondere zur Verwendung mit Einweg-Injektionsgeräten an, alternativ dazu kann ein elektronisches, insbesondere Mehrweg-, Injektionsgerät auch selbst mit geeigneten Sensoren und einem elektrischen Energiespeicher zur Speisung der Sensoren und weiterer elektronischer Komponenten ausgestattet sein.

Zur Optimierung der Energieversorgung und insbesondere zur Minimierung der Kapazität des Energiespeichers wird das Zusatzmodul oder das elektronische Injektionsgerät wenn immer möglich in einem Energiesparmodus oder Schlafmodus betrieben, und Funktionen mit erhöhtem Energieverbrauch werden nur selektiv aktiviert oder aufgeweckt. Das Aktivieren dieser Funktionen kann erfolgen durch einen elektromechanischen Schalter, welcher durch den Anwender im richtigen Zeitpunkt direkt betätigt oder zum Beispiel durch Entfernen einer die Kanüle oder Hohlnadel schützenden Gerätekappe indirekt ausgelöst werden muss. Diese Vorgänge setzen eine bewusste Manipulation durch den Anwender voraus. Diese kann aber vergessen gehen oder, falls beispielsweise vorgängig die Gerätekappe nicht korrekt aufgesetzt wurde, aus anderen Gründen unentdeckt bleiben. Geeignete Sensoren, beispielsweise kapazitive Berührungssensoren an der Oberfläche des Zusatzmoduls oder Injektionsgeräts, können das Ergreifen und/oder Bewegen desselben durch den Anwender detektieren.

Die Patentanmeldung WO18104289 beschreibt ein Zusatzmodul für ein Injektionsgerät mit variabler Dosiseinstellung, welches an einen Dosiseinstellknopf am proximalen Ende des Injektionsgeräts aufgesetzt wird. Ein Schaltmechanismus umfasst eine erste Komponente am Gehäuse des Zusatzmoduls und eine zweite Komponente welche permanent im oder am Gehäuse eines jeden Injektionsgeräts vorgesehen ist. Beim Drehen des Dosiseinstellknopfs relativ zum Gehäuse des Injektionsgeräts werden die zwei Komponenten getrennt und der Schaltmechanismus geöffnet, worauf weitere elektronische Komponenten des Zusatzmoduls aktiviert werden.

Die Patentanmeldung WO18013419 beschreibt ein Zusatzmodul für ein Injektionsgerät mit variabler Dosiseinstellung, welches an einen Dosiseinstellknopf am proximalen Ende des Injektionsgeräts aufgesetzt wird. Das Zusatzmodul umfasst zwei axial relativ zueinander bewegliche und durch eine Feder getrennte Modulteile. Durch eine axiale Lösebewegung wird die Feder komprimiert, wodurch ein elektrischer Kontakt geschlossen und eine Prozessoreinheit des Zusatzmoduls aktiviert wird.

WO 2020058875 A1 offenbart ein Zusatzmodul für ein Injektionsgerät, wobei das Zusatzmodul ein Sensorelement zur Detektion eines Zustandes oder Vorganges im Injektionsgerät umfasst. Das Zusatzmoduls umfasst ein mehrteilig ausgebildetes Gehäuse, einen Gehäuseeinsatz und eine Aufnahmeeinheit. Diese ist im Gehäuse axial beweglich gelagert und umfasst Sensorelemente in Form von zwei induktiven Sensorspulen. Ein auf einer Leiterplatine des Zusatzmoduls angeordneter Schalter detektiert das Ende der Aufnahmebewegung der Aufnahmeeinheit. Ein allfälliges Fehlen des Injektionsgerätes kann über die aktivierten Sensorelemente oder ein sonstiges RF-Identifikationssignal des Injektionsgerätes festgestellt werden.

WO18013419 A1 offenbart ein Zusatzmodul zum Erkennen einer eingestellten Dosis. Das Zusatzmodul wird auf ein proximalen Ende eines Injektionspens aufgesetzt. Ein Verbindungselement des Zusatzmoduls umfasst einen kreisrunden Bereich, welcher ein Dosierelement umschliesst, um das Zusatzmodul am Pen zu befestigen. Zum Ausschütten drückt der Benutzer in axialer Richtung auf das Zusatzmodul und diese axiale Kraft wird über das Zusatzmodul auf einen Aktuator des Pens übertragen, so dass ein Ausschüttknopf des Pens betätigt wird und dadurch der Aktuator rotativ vom Dosierelement freigegeben wird. Dadurch schraubt sich das Dosierelement zurück in das Pengehäuse.

Der Begriff "Medikament" oder "medizinische Substanz" umfasst in diesem Zusammenhang jede fliessfähige medizinische Formulierung, welche geeignet ist zur kontrollierten Verabreichung mittels einer Kanüle oder Hohlnadel, beispielsweise eine Flüssigkeit, eine Lösung, ein Gel oder eine feine Suspension enthaltend einen oder mehrere medizinische Wirkstoffe. Ein Medikament kann also eine Zusammensetzung mit einem einzigen Wirkstoff oder eine vorgemischte oder coformulierte Zusammensetzung mit mehreren Wirkstoffen aus einem einzelnen Behälter sein. Der Begriff umfasst insbesondere Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form. Der Begriff umfasst weiter auch Polysaccharide, Vakzine, DNS oder RNS oder Oligonukleotide, Antikörper oder Teile von Antikörpern sowie geeignete Basis-, Hilfs- und Trägerstoffe.

### DARSTELLUNG DER ERFINDUNG

Es ist eine Aufgabe der Erfindung ein elektronisches Zusatzmodul für ein Injektionsgerät anzugeben, welches eine durch den Anwender vorgängig der Ausschüttung vorzunehmende Manipulation am Zusatzgerät zuverlässig detektieren und dadurch eine Funktion zur Charakterisierung der Ausschüttung selektiv aktivieren kann. Die Erfindung ist definiert durch den Gegenstand von Anspruch 1. Weitere Ausführungsformen sind in den abhängigen Ansprüchen angegeben.

Ein elektronisches Zusatzmodul nach der Erfindung wird auf ein Injektionsgerät in Richtung einer Längsachse, welche ein einstechseitiges distales Ende mit einem gegenüberliegenden proximalen Ende des Injektionsgeräts verbindet, lösbar aufgesetzt oder aufgeschoben, beziehungsweise das Injektionsgerät wird in einen Aufnahmebereich des aufgeschoben, beziehungsweise das Injektionsgerät wird in einen Aufnahmebereich des Zusatzmoduls eingeführt oder eingeschoben. Das Zusatzmodul ist im Allgemeinen hülsenförmig ausgebildet und der Aufnahmebereich weist eine an den Querschnitt des Injektionsgerätes angepasste Öffnung auf, so dass im aufgesetzten Zustand das Injektionsgerät nicht quer zur Längsachse aus dem Aufnahmebereich entfernt werden oder gar fallen kann. Das Injektionsgerät umfasst ein Gerätegehäuse welches eine Abgabemechanik zur parenteralen subkutanen oder intramuskulären Abgabe von Medikamenten aus einem Behälter des Injektionsgerätes umgibt. Das Injektionsgerät verfügt an seinem proximalen Ende über einen Ausschüttknopf, welcher durch einen Anwender in distale Richtung bewegbar ist und dadurch eine Ausschüttung bewirkt oder auslöst. Das Zusatzmodul umfasst eine Sensoreinheit zur Erkennung von Vorgängen oder Zuständen im Injektionsgerät, sowie eine Prozessoreinheit zur Auswertung und/oder Verarbeitung eines Signals der Sensoreinheit. Das Zusatzmodul umfasst weiter einen Energiespeicher zur Energieversorgung von Sensor- und/oder Prozessoreinheit, und eine Kommunikationseinheit zur drahtlosen Kommunikation von Signalen der Sensoreinheit und/oder von Daten der Prozessoreinheit. Der Aufnahmebereich des Zusatzmoduls umfasst das Injektionsgerät im eingeschobenen Zustand derart, dass der Ausschüttknopf durch den Anwender unmittelbar berührt und bedient werden kann, und somit nicht durch das Zusatzmodul umgeben oder sonst wie abgedeckt ist.

Erfindungsgemäss umfasst das Zusatzmodul eine erste und eine zweite Modulkomponente, welche aus einem Ausgangszustand des Injektionssystems durch eine Aktion des Anwenders und gegen die Wirkung eines Federelements relativ gegeneinander in einer Bewegungsrichtung bewegt werden können. Die erste Modulkomponente umfasst ein Modulgehäuse beziehungsweise zumindest einen an einer äusseren Oberfläche des Modulgehäuses gebildeten Griff zum Ergreifen und Halten des Zusatzmoduls durch den Anwender, und die zweite Modulkomponente ist in Bewegungsrichtung an das Gerätegehäuse gekoppelt oder lösbar daran fixiert. Das Zusatzmodul umfasst weiter einen Bewegungs- oder Positionsdetektor zur Detektion dieser Relativbewegung der beiden Modulkomponenten. Die Sensoreinheit ist so ausgebildet und angesteuert, dass sie aus einem ersten Betriebsmodus nach Detektion einer Relativbewegung der Modulkomponenten in einen zweiten Betriebsmodus wechselt oder übergeht. In bestimmten Zuständen des Injektionssystems und insbesondere nach längerer Inaktivität wird eine detektierte Relativbewegung durch eine Prozessoreinheit des Zusatzmoduls als Aktion des Anwenders zur Vorbereitung einer Ausschüttung identifiziert. Die Sensoreinheit wird dann aus dem ersten Betriebsmodus, beispielsweise einem stromlosen Lagerzustand oder einem Schlafzustand mit reduziertem Energieverbrauch pro Zeiteinheit, eingeschaltet beziehungsweise geweckt, und/oder es werden gewisse Funktionen der Sensoreinheit zur Beobachtung einer erwarteten Ausschüttung aktiviert.

Im Ausgangszustand werden die zwei Modulkomponenten durch das Federelement derart zueinander positioniert, dass nach Überschreiten einer gewissen Kraftschwelle ein kleiner Hub in Bewegungsrichtung und dadurch ein Auslösen des Detektors möglich ist. Die Kraftschwelle ist beispielsweise definiert durch eine Vorspannung eines linearen Federelements, durch eine nichtlineare Federkennlinie, und/oder durch zusätzliche Haftreibung zwischen den Modulkomponenten. Die Relativbewegung kann durch den Anwender ebenfalls wahrnehmbar sein, ihr Hub oder ihre Amplitude übertrifft toleranzbedingte Ungenauigkeiten in der Ausrichtung von Zusatzmodul und Injektionsgerät, beträgt aber gleichzeitig weniger als 3 mm, bevorzugt weniger als 1 mm, in axialer Richtung oder weniger als 15°, bevorzugt weniger als 5°, in Drehrichtung. Der Detektor detektiert die Relativbewegung oder die relative Anfangs- oder Endposition der beiden Modulkomponenten zueinander. Bevorzugt wird durch das Federelement die eine Modulkomponente in einem Anschlag mit der zweiten Modulkomponente gehalten, so dass die Relativbewegung nur in einer Detektionsrichtung möglich ist. Alternativ dazu kann die eine Modulkomponente in zwei entgegengesetzte Detektionsrichtungen relativ zur zweiten Modulkomponente bewegt werden, sie ist dazu beispielsweise durch zwei annähernd gleich starke Federelemente in einer Mitteposition gehalten.

Der Detektor kann auf jeder der beiden Modulkomponenten angeordnet sein, und mit einem geeigneten Gegenpart auf der jeweils anderen Modulkomponente zusammenwirken, beispielsweise mit einer mechanischen Kontaktfläche im Falle eines Piezodetektors oder mit einer optischen Blende bei Einsatz einer Lichtschranke. Bevorzugt ist der Detektor ein elektronischer Mikroschalter oder ein elektromechanischer Schalter, bei welchem durch Bewegen eines Kontaktelements oder Kippen eines Schalthebels ein Kontakt geschlossen wird, was durch ein Prozessorelement erkannt und zur Aktivierung der Sensoreinheit genutzt werden kann.

Die zweite Modulkomponente ist zumindest in Richtung der zu detektierenden Relativbewegung zum oder am Gerätegehäuse des Injektionsgeräts fixiert, so dass die zweite Modulkomponente eine entsprechende Bewegung des Injektionsgeräts ungedämpft und verzögerungsfrei mitmacht. Die zweite Modulkomponente kann mittels Kraftschluss flächig auf das Gerätegehäuse gepresst sein oder mittels Formschluss an Aus- oder Einprägungen des Injektionsgerätes angreifen. Insbesondere sind diese Aus- oder Einprägungen an einem Bereich des Injektionsgeräts vorgesehen, welcher durch eine Gerätekappe nicht abgedeckt wird, so dass die Gerätekappe auch bei aufgesetztem Zusatzmodul auf das Injektionsgerät aufgeschoben oder von diesem abgezogen werden kann.

Die erste Modulkomponente ist zum oder am Modulgehäuse fixiert, insbesondere Teil eines Haltegriffs, durch welchen der Anwender das Zusatzmodul mitsamt dem gehaltenen Injektionsgerät ergreifen kann. Der Haltegriff weist dazu in Längsrichtung eine Ausdehnung auf, welche mindestens einer halben Handbreite des Anwenders entspricht, spezifisch mindestens 3 cm breit ist, und eine zum Halten in Längsrichtung geeignet gestaltete Oberfläche umfassen kann. Der Haltegriff ist derart ausgestaltet dass ein Anwender bei Ergreifen des Griffs mit den Fingern einer ersten Hand alle relevanten Manipulationen mit dem Injektionssystem ausführen kann, beispielsweise Aufsetzen auf der Injektionsstelle, Einstechen, und Ausschütten durch Berühren des Ausschüttknopfs mit dem Daumen der ersten Hand. Mit Hilfe der zweiten Hand können weitere Manipulationen wie Entfernen der Gerätekappe, Montage einer Injektionsnadel, und Einstellen einer Dosis vorgenommen werden. Die zweite Modulkomponente ist radial zwischen dem Haltegriff und der Geräteaufnahme vorgesehen.

In einer bevorzugten Ausführungsform umfasst die Sensoreinheit einen Sensor zur optischen, mechanischen oder elektrischen, insbesondere magnetischen oder induktiven, Erkennung von Vorgängen oder Zuständen im Injektionsgerät. Der Sensor erzeugt ein Sensorsignal, welches durch einen A/D Wandler abgetastet und mit einer bestimmten Datenrate von der Sensoreinheit bereitgestellt und durch die Prozessoreinheit weiter verarbeitet wird. Der Sensor und/oder der A/D Wandler, insbesondere eine Empfindlichkeit des Sensors und/oder eine Abtastfrequenz des A/D Wandlers, werden erst nach Detektion der besagten Relativbewegung in den für die verlässliche Erkennung der Vorgänge oder Zustände geeigneten zweiten Betriebsmodus geschaltet. Durch einen optimierten Start und eine zeitliche Eingrenzung dieses Betriebsmodus auf die Dauer einer Ausschüttung ist der zugehörige Energieverbrauch pro Zeiteinheit und/oder ein der bereitgestellten Datenrate entsprechender Datenspeicherbedarf pro Zeiteinheit gesamthaft verkraftbar, und kann insbesondere höher sein als in einem zeitlich nicht oder weniger eingeschränkten Dauer- oder Standardbetriebsmodus, ohne dass dazu eine Energie- oder Datenspeichereinheit grosszügiger dimensioniert werden muss.

Optional umfasst das Zusatzmodul auch einen Schalter welcher bei Aufschieben des Zusatzmoduls durch das Injektionsgerät oder beim Einstecken eines Ladekabels durch einen Stecker des Ladekabels betätigt wird und elektronische Komponenten des Zusatzmoduls aus einem stromlosen Lagerzustand zumindest in einen Schlafmodus versetzt. Durch einen zusätzlichen Gerätekappendetektor und/oder einen Berührungssensor kann eine erwartete Ausschüttung noch eindeutiger identifiziert werden. Parallel zum zweiten Betriebsmodus der Sensoreinheit können zu Beginn eines erwarteten Injektionsvorgangs auch weitere Funktionen des Zusatzmoduls wie ein User-Interface oder die Kommunikationseinheit aktiviert oder auf einen zweiten Modus gewechselt werden.

In einer bevorzugen Ausführungsform ist die Relativbewegung zwischen den Modulkomponenten ausschliesslich axial, in einer Detektionsrichtung parallel zur Längsachse, zum Beispiel beim Abzug der Schutzkappe des Injektionsgeräts oder beim Drücken auf den proximalen Ausschüttknopf, sowie beim Aufsetzen der Schutzkappe des Injektionsgeräts, bei der Montage einer Injektionsnadel, oder beim Anpressen des Injektionsgeräts auf die Injektionsstelle. Im ersten Fall wird die zweite Modulkomponente relativ zur ersten Modulkomponente Richtung distal bewegt, und im zweiten Fall Richtung proximal. Alternativ kann die Relativbewegung auch radial sein, zum Beispiel bei einer rotativen Dosierbewegung mittels eines Dosierknopfs, speziell zu einem Nullpunktanschlag hin.

In einer bevorzugten Ausführungsform umfasst die Sensoreinheit mindestens einen ein- oder mehrachsigen Beschleunigungssensor, einen ein- oder mehrachsigen Gyroskopsensor, einen Trägheitssensor, ein piezobasiertes Körperschallmikrofon, oder einen vergleichbaren Sensor zur Erkennung von Bewegungen des Injektionsgerätes. Diese Bewegungen umfassen dabei sowohl Bewegungen des Injektionsgeräts im Raum, welche durch einen Anwender ausgeführt werden, als auch Bewegungen des Injektionsgeräts, welche durch einen Mechanismus im Injektionsgerät verursacht werden, beispielsweise Vibrationen, Schwingungen oder Schallwellen von Ausschüttklicks welche im Injektionsgerät während oder am Ende der Ausschüttung einer Dosis erzeugt werden. Dazu liegt im aufgesetzten Zustand eine Begrenzungsfläche des Aufnahmebereichs an einer Auflagefläche des Injektionsgeräts zumindest radial möglichst dicht an. Die Sensoreinheit ist bevorzugt Teil der zweiten Modulkomponente, so dass neben den zu detektierenden Bewegungen des Injektionsgerätes als Ganzes gleichzeitig auch die Vibrationen in axialer Richtung an die zweite Modulkomponente übertragen und von der Sensoreinheit erkannt werden können. Dementsprechend ist die Sensoreinheit nicht ausgebildet zum Lesen einer angezeigten Dosis, eine axiale Ausdehnung des Zusatzmoduls ist bevorzugt so gewählt, dass ein Dosisanzeigefenster des Injektionsgeräts zur Anzeige einer eingestellten Dosis vom aufgesetzten Zusatzmodul nicht überdeckt wird und für den Anwender die Dosisanzeige unmittelbar sichtbar bleibt.

In einer vorteilhaften Variante der Erfindung ist der Aufnahmebereich zur Aufnahme des Injektionsgeräts durch Einschieben des Injektionsgeräts in distale Richtung beziehungsweise durch Aufschieben des Zusatzmoduls auf das Injektionsgerät in proximaler Richtung ausgebildet. Das Injektionsgerät umfasst eine nach distal gerichtete Stopp- oder Anschlagfläche zur Begrenzung der Aufsetzbewegung des Zusatzmoduls auf das Injektionsgerät in proximaler Richtung. Diese Stoppfläche wird bevorzugt beim Übergang zwischen einem distalen Karpulenhalter und einem proximalen Gerätegehäuse des Injektionsgeräts gebildet. Ein Dosiswahlknopf, und optional auch eine Dosisanzeige, am proximalen Ende des Injektionsgerätes wird dabei nicht durch das Zusatzmodul berührt oder umfasst, so dass der Aufnahmebereich nicht auf die Dimension des Dosiswahlknopf abgestimmt sein muss und dieser somit eine grössere Ausdehnung senkrecht zur Längsachse aufweisen kann als das Gerätegehäuse des Injektionsgerätes. Die erste Modulkomponente umfasst ein Löseelement mit einem Halteelement, welches in einer Verriegelungsbewegung eine nach proximal gerichteten Haltefläche des Injektionsgeräts hintergreift und das Injektionsgerät gegen eine Trennbewegung nach proximal hält und verriegelt.

In einer bevorzugten Weiterentwicklung umfasst die zweite Modulkomponente ein Steuerelement welches bei Einschub des Injektionsgeräts unter Spannung beziehungsweise gegen die Wirkung des Federelements bewegt wird. Die Einschubbewegung erfolgt in Detektionsrichtung und bewirkt eine Vorspannung des Federelements zwischen dem Modulgehäuse und einem Halteelement der ersten Modulkomponente. Die Haltefläche des Injektionsgeräts ist Teil einer Ausprägung, welche deutlich gegenüber einer sie umgebenden Oberfläche des Injektionsgeräts abweicht, das Steuerelement in Einschub- und Detektionsrichtung formschlüssig mitbewegt, und durch das Federelement zusammen mit dem Steuerelement axial festgeklemmt wird. Diese Bewegung des Steuerelements gibt eine Verriegelungsbewegung des Löseelements frei, bei welcher das Halteelement in Eingriff mit der Haltefläche an der Ausprägung des Injektionsgeräts bewegt und das Zusatzmodul mit dem Injektionsgerät verriegelt wird.

In einer bevorzugten Weiterentwicklung erfolgt die Verriegelungsbewegung automatisch durch eine Rückstellfeder, welche über die axiale Einschubbewegung des Injektionsgeräts entsperrt wird. Durch die Rückstellfeder kann reproduzierbar ein deutlich hörbarer Verriegelungsklick als Haltezustands-Rückmeldung an den Anwender generiert werden. Die Rückstellfeder ist Teil des Federelements, wobei auf die Spannung des Federelements in Einschubrichtung eine freigegebene Entspannung des Federelements und Verriegelungsbewegung des Löseelements in Verriegelungsrichtung folgt.

Das Injektionsgerät selbst verfügt nicht über die Fähigkeit zur Kommunikation mit einem weiteren Gerät, kann aber durchaus zur Wechselwirkung mit dem Zusatzgerät angepasst sein, beispielsweise durch geeignete Öffnungen im Gerätegehäuse oder durch bewegliche Aktoren wie Magnete oder Schleifkontakte, deren Positionen durch den Sensor des Zusatzmoduls deutlich erkannt werden können. Das Injektionsgerät es ist bevorzugt ein Einweg-Injektionsgerät ohne die Möglichkeit eines Ersatzes des Behälters durch einen Anwender, oder auch ein Mehrweg-Injektionsgerät. Das Injektionsgerät ist weiter bevorzugt ein Injektionsgerät mit variabler Dosiseinstellung, umfassend einen am proximalen Ende angeordneten Dosiseinstellknopf zum Einstellen einer auszuschüttenden Dosis mittels einer Dreh- und/oder Schraubbewegung. Das Injektionsgerät kann auch zur einmaligen Abgabe einer fixen Dosis vorgesehen sein. Das Injektionsgerät wird entweder durch Übertragen einer Ausschüttkraft des Anwenders auf den Ausschüttknopf manuell betrieben, oder es umfasst eine vorgespannte Feder welche durch Betätigen des Ausschüttknopfs für eine automatische Ausschüttung freigegeben wird.

Die Erfindung ist auch auf Injektionsgeräte ohne proximalen Auslöse- oder Ausschüttknopf anwendbar, beispielsweise sogenannte 2-Schritt Autoinjektoren, welche durch Kontakt mit der Injektionsstelle die Ausschüttung auslösen. In diesem Fall ist zumindest der Abzug einer Nadelschutzkappe oder das Aufsetzen auf die Injektionsstelle detektierbar.

Es ist eine weitere Aufgabe der Erfindung ein elektronisches Injektionsgerät anzugeben, welches eine durch den Anwender vorgängig der Ausschüttung vorzunehmende Manipulation zuverlässig detektieren und dadurch eine Funktion zur Charakterisierung der Ausschüttung selektiv aktivieren kann. Diese Aufgabe wird gelöst durch ein elektronisches Injektionsgerät mit einer Dosiervorrichtung zur Ausschüttung von einstellbaren Dosen eines flüssigen Medikaments aus einem Behältnis des Injektionsgeräts, in welches die Sensoreinheit des vorgängig beschriebenen Zusatzmoduls zur optischen, mechanischen oder elektrischen Erkennung von Vorgängen oder Zuständen im Injektionsgerät, sowie ein elektrischer Energiespeicher zur Speisung der Sensoreinheit und weiterer elektronischer Komponenten vollständig integriert sind. Das Injektionsgerät umfasst eine erste Gerätekomponente mit einem Griff zum Ergreifen und Halten des Injektionsgeräts durch den Anwender, eine zweite Gerätekomponente, welche durch eine Aktion des Anwenders und unter Spannung eines Federelements in einer Bewegungsrichtung relativ zur ersten Gerätekomponente bewegt werden kann, und einen Detektor zur Detektion dieser Relativbewegung, wobei die Sensoreinheit nach Detektion einer Relativbewegung der Gerätekomponenten von einem ersten in einen zweiten Betriebsmodus wechselt.

Das Injektionsgerät umfasst ein Gerätegehäuse welches eine Abgabemechanik zur parenteralen subkutanen oder intramuskulären Abgabe von Medikamenten aus einem Behälter des Injektionsgerätes umgibt. Das Injektionsgerät umfasst eine Karpulenhalter zur Aufnahme des Behälters, eine Dosiervorrichtung zur Einstellung einer auszuschüttenden Dosis, eine Sensoreinheit zur Erkennung von Vorgängen oder Zuständen im Injektionsgerät, sowie eine Prozessoreinheit zur Auswertung und/oder Verarbeitung eines Signals der Sensoreinheit. Das Injektionsgerät umfasst weiter einen Energiespeicher zur Energieversorgung von Sensor- und/oder Prozessoreinheit, und eine Kommunikationseinheit zur drahtlosen Kommunikation von Signalen der Sensoreinheit und/oder von Daten der Prozessoreinheit. Das Injektionsgerät verfügt bevorzugt an seinem proximalen Ende über einen Ausschüttknopf, welcher durch einen Anwender in distale Richtung bewegbar ist und dadurch eine Ausschüttung bewirkt oder auslöst.

Das Injektionsgerät umfasst eine erste und eine zweite Gerätekomponente, welche aus einem Ausgangszustand des Injektionssystems durch eine Aktion des Anwenders und gegen die Wirkung eines Federelements relativ gegeneinander in einer Bewegungsrichtung bewegt werden können. Die erste Gerätekomponente umfasst ein Gerätegehäuse beziehungsweise zumindest einen an einer äusseren Oberfläche des Gerätegehäuses gebildeten Griff zum Ergreifen und Halten des Injektionsgeräts durch den Anwender. Die zweite Gerätekomponente ist in Bewegungsrichtung vom Gerätegehäuse entkoppelt und separat durch eine durch den Anwender vorgängig der Ausschüttung vorzunehmende Manipulation gegenüber der ersten Gerätekomponente bewegbar. Die zweite Gerätekomponente ist beispielsweise ein Karpulenhalter mit einem distalen Ende zum Aufschrauben oder Aufschnappen einer Nadeleinheit mit einer Injektionsnadel, und/oder mit einer proximalen Gerätekappenhalterung in Form eines Nockens oder eines Schnappers am Karpulenhalter zum Eingriff in einen Schnapper oder Nocken an der Gerätekappe. Die zweite Gerätekomponente kann ein proximaler Ausschüttknopf sein, welcher durch einen Anwender in distale Richtung bewegbar ist und dadurch eine Ausschüttung bewirkt oder auslöst. Die zweite Gerätekomponente kann ein Dosiswahlknopf sein, welcher zur Einstellung oder Kalibrierung der Nullposition für die nachfolgende Dosiswahl beziehungsweise für die Erkennung derselben durch den Anwender in die Nullposition gedrängt wird.

Das Injektionsgerät umfasst weiter einen Bewegungs- oder Positionsdetektor zur Detektion dieser Relativbewegung der beiden Gerätekomponenten, verursacht durch Aufsetzen oder Entfernen einer Nadeleinheit oder einer Gerätekappe auf den beziehungsweise vom Karpulenhalter, durch Einstechen der Nadel in eine Injektionsstelle, oder durch Betätigen des Ausschüttknopfs oder des Dosiswahlknopfs. Die Sensoreinheit ist so ausgebildet und angesteuert, dass sie aus einem ersten Betriebsmodus nach Detektion einer Relativbewegung der Gerätekomponenten in einen zweiten Betriebsmodus wechselt oder übergeht. In bestimmten Zuständen des Injektionsgeräts und insbesondere nach längerer Inaktivität wird eine detektierte Relativbewegung durch eine Prozessoreinheit des Injektionsgeräts als Aktion des Anwenders zur Vorbereitung einer Ausschüttung identifiziert. Die Sensoreinheit wird dann aus dem ersten Betriebsmodus, beispielsweise einem stromlosen Lagerzustand oder einem Schlafzustand mit reduziertem Energieverbrauch pro Zeiteinheit, eingeschaltet beziehungsweise geweckt, und/oder es werden gewisse Funktionen der Sensoreinheit zur Beobachtung einer erwarteten Ausschüttung aktiviert. Parallel zum zweiten Betriebsmodus der Sensoreinheit können zu Beginn eines erwarteten Injektionsvorgangs auch weitere Funktionen wie ein User-Interface oder die Kommunikationseinheit aktiviert oder auf einen zweiten Modus gewechselt werden. Optional umfasst das Injektionsgerät auch einen Schalter welcher durch den Anwender bei erstmaliger Inbetriebnahme oder beim Einstecken eines Ladekabels durch einen Stecker des Ladekabels betätigt wird und elektronische Komponenten des Injektionsgeräts aus einem stromlosen Lagerzustand zumindest in einen Schlafmodus versetzt.

Im Ausgangszustand werden die zwei Gerätekomponenten durch das Federelement derart zueinander positioniert, dass nach Überschreiten einer gewissen Kraftschwelle ein kleiner Hub in Bewegungsrichtung und dadurch ein Auslösen des Detektors möglich ist. Die Relativbewegung kann durch den Anwender ebenfalls wahrnehmbar sein, ihr Hub oder ihre Amplitude übertrifft toleranzbedingte Ungenauigkeiten in der Ausrichtung der zwei Gerätekomponenten, beträgt aber gleichzeitig weniger als 3 mm, bevorzugt weniger als 1 mm, in axialer Richtung oder weniger als 15°, bevorzugt weniger als 5°, in Drehrichtung. Der Detektor detektiert die Relativbewegung oder die relative Anfangs- oder Endposition der beiden Gerätekomponenten zueinander. Bevorzugt wird durch das Federelement die eine Gerätekomponente in einem Anschlag mit der zweiten Gerätekomponente gehalten, so dass die Relativbewegung nur in einer Detektionsrichtung möglich ist.

Für den Fachmann sind direkt und offensichtlich weitere Ausführungsformen und Ausgestaltungen erkennbar, welche sich aus Kombinationen der beschriebenen Beispiele oder Kombinationen der beschriebenen Beispiele mit dem allgemeinen Fachwissen des Fachmanns ergeben. Insbesondere sind die bevorzugten Ausführungsformen des Injektionssystems auch auf das elektronische Injektionsgerät anwendbar.

### FIGUREN

In Zusammenhang mit den angehängten Figuren werden nachfolgend bevorzugte Ausführungsformen der Erfindung beschrieben. Diese sollen grundsätzliche Möglichkeiten der Erfindung aufzeigen und keinesfalls einschränkend ausgelegt werden. Es zeigen
Fig.1 ein Injektionsgerät mit variabler Dosisabgabe;
Fig.2 zwei Injektionsgeräte mit je einem aufgesetzten Zusatzmodul nach der Erfindung;
Fig.3 ein Injektionsgerät mit einem aufgesetztem Zusatzmodul mit Steuerelement; und
Fig.4 ein Injektionsgerät mit einem aufgesetztem Zusatzmodul mit Rückstellfeder.

### FIGURENBESCHREIBUNG

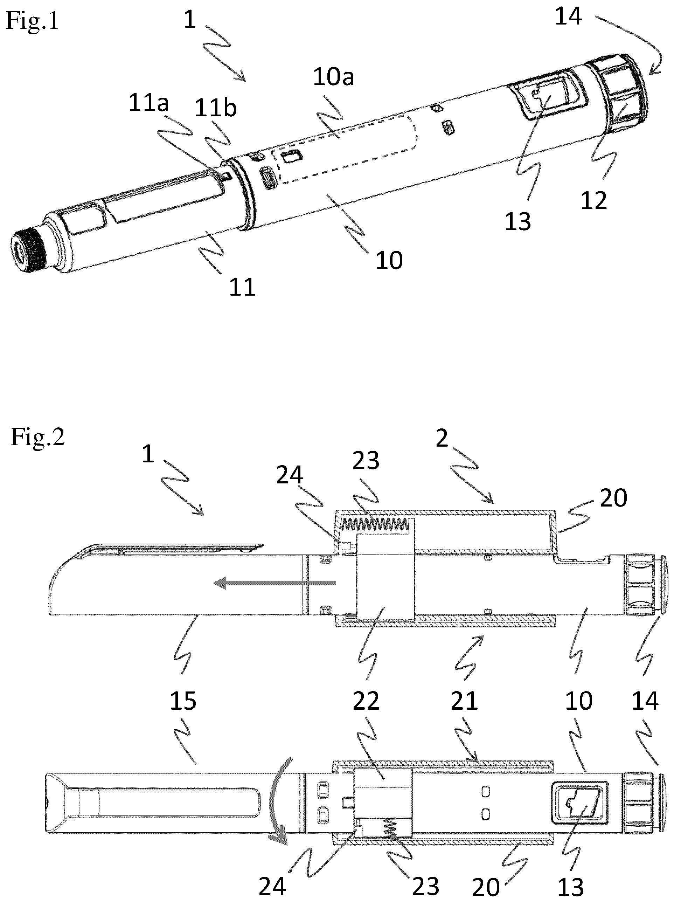

Fig.1 zeigt in Schrägaufsicht ein Injektionsgerät 1 mit variabler Dosisabgabe, umfassend ein längliches Gerätegehäuse 10 mit einer Längsachse, einen Karpulenhalter 11, einen Dosiswahlknopf 12, eine Dosisanzeige 13, und einen Ausschüttknopf 14. Der Karpulenhalter 11 weist einen Nocken 11a auf sowie eine ringförmige Stoppfläche 11b beim Übergang zum Gerätegehäuse 10. Ein distales Ende des Karpulenhalters 11 (links in Fig.1) weist ein Gewinde auf zum Aufbringen einer Nadeleinheit mit einer Injektionsnadel. Eine in Fig.1 nicht gezeigte Geräte- oder Nadelschutzkappe des Injektionsgeräts kann in einer den Karpulenhalter 11 abdeckenden Position mit dem Nocken 11a eine Schnappverbindung eingehen. Eine mögliche Ausdehnung einer Auflagefläche 10a auf der Oberseite des Gerätegehäuses ist mit einer unterbrochenen Linie skizziert. Auf einer der Auflagefläche 10a gegenüberliegender, in Fig.1 nicht sichtbarer Unterseite des Gerätegehäuses kann eine Etikette des Injektionsgerätes aufgebracht sein.

Fig.2 zeigt eine Ansicht (oben) und eine Aufsicht (unten) des Injektionsgeräts mit Gerätegehäuse 10 und aufgesetzter Gerätekappe 15, und jeweils einen Längsschnitt durch ein aufgesetztes Zusatzmodul 2. Jedes Zusatzmodul umfasst ein Modulgehäuse 20, ein elastisches Element in Form eines Federelements 23, und einen Detektor 24. Eine erste Modulkomponente 21 umfasst das Modulgehäuse 20 beziehungsweise zumindest einen an einer äusseren Oberfläche des Modulgehäuses gebildeten Griff zum Ergreifen und Halten des Zusatzmoduls durch mindestens zwei Finger einer Hand eines Anwenders. Eine zweite Modulkomponente 22 ist am Gerätegehäuse 10 derart fixiert, dass sie eine Bewegung des Injektionsgeräts relativ zur ersten Modulkomponente in eine Detektionsrichtung (Pfeil) ohne Verzögerung mitmacht. Das Federelement 23 drängt die beiden Modulkomponenten entgegen der Detektionsrichtung auseinander beziehungsweise in eine Anschlagsposition. Der Detektor 24 ist als elektromechanischer Schalter ausgebildet und detektiert die Bewegung des Injektionsgeräts relativ zur ersten Modulkomponente in Detektionsrichtung, bei welcher das Federelement 23 um einen kleinen Hub komprimiert wird. In Fig.2 oben ist diese Bewegung eine lineare Bewegung in distaler Richtung, beispielsweise verursacht durch einen Abzug der Gerätekappe 15 oder durch Drücken des Ausschüttknopfs 14. Falls nur die Ausschüttkraft aber nicht die Kappenabzugskraft grösser ist als eine Federkraft des Federelements 23 kann dabei auch selektiv nur die Ausschüttung detektiert werden. In Fig.2 unten ist diese Bewegung eine Drehbewegung im Gegenuhrzeigersinn (Blickrichtung distal), beispielsweise dadurch verursacht, dass die Dosisanzeige 13 beziehungsweise der Dosiswahlknopf in eine Nullposition gedreht werden, was zur Einstellung oder Kalibrierung der Nullposition für die nachfolgende Dosiswahl beziehungsweise für die Erkennung derselben durch das Zusatzmodul erforderlich sein kann.

Die zweite Modulkomponente 22 kann dazu in Form einer Klammer kraftschlüssig an eine Oberfläche des Gerätegehäuses 10 angepresst sein und sämtliche Axial- wie Drehbewegungen des Injektionsgeräts mit diesem zusammen ausführen. Die zweite Modulkomponente kann auch formschlüssig über senkrecht zur Detektionsrichtung ausgebildete Halteflächen mit Ein- oder Ausprägungen am Injektionsgerät zusammenwirken, wobei eine Bewegung entgegengesetzt zur Detektionsrichtung nicht auf die zweite Modulkomponente übertragen wird. In der Ausführungsform von Fig.2 unten ist das Injektionsgerät beispielsweise durch Ausprägungen seiner Oberfläche, welche in axialen Rillen oder Nuten in einer Begrenzungsfläche des Aufnahmebereichs des Zusatzmoduls geführt sind, in der zweiten Modulkomponente drehfest aufgenommen.

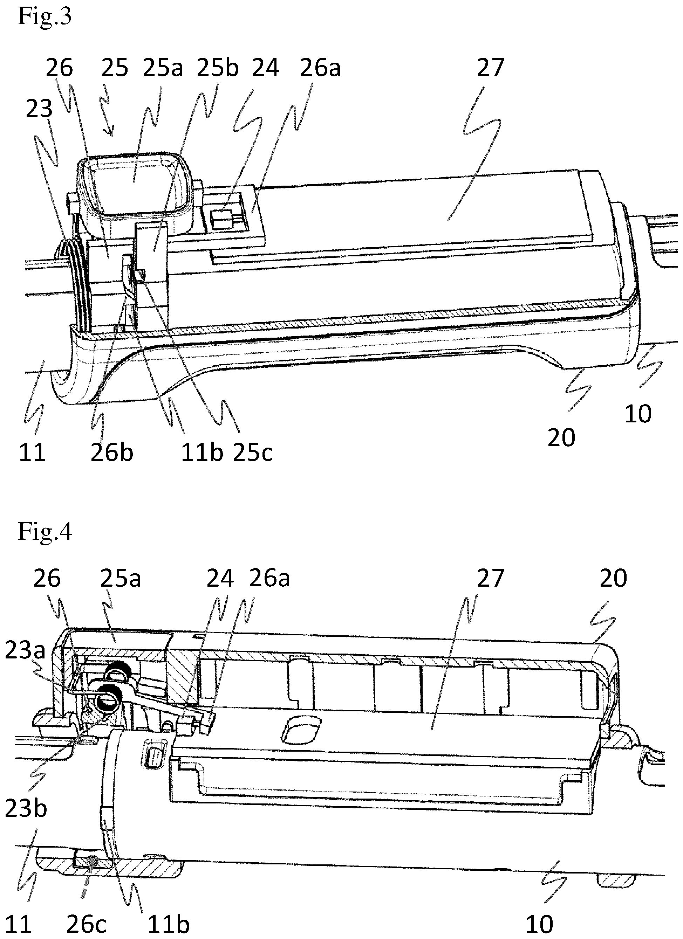

Fig.3 zeigt eine Schrägaufsicht eines Teils eines Injektionsgeräts 1 mit Gerätegehäuse 10 und Karpulenhalter 11 sowie ein auf das Injektionsgerät aufgesetztes und mit diesem in einem Haltezustand verriegeltes Zusatzmodul 2. Eine obere Hälfte des Modulgehäuses 20 wurde entfernt und gibt den Blick frei auf ein Löseelement 25 in Form eines Druckknopfes mit einer nach oben gerichteten Bedienfläche 25a und zwei nach unten gerichteten parallelen Armen, wobei in der gewählten Ansicht nur der dem Betrachter zugewandte Arm 25b sichtbar ist. An Stelle des Druckknopfes kann auch ein Schieber in einer Richtung quer zur Längsrichtung als Löseelement gewählt werden. Entsprechende und weitere Ausführungsformen sind in der Patentanmeldung mit dem Anmeldeaktenzeichen PCT/EP2021/059606 ausführlich beschrieben.

Das Löseelement 25 wird durch eine Rückstellfeder (nicht erkennbar) nach oben vorgespannt, und durch Druck auf die Bedienfläche 25a zur Lösung der Verriegelung nach unten bewegt. Ein axial bewegliches Steuerelement 26 ist ringförmig ausgebildet und wird vom eingeschobenen Injektionsgerät durchdrungen. Ein Federelement 23 in Gestalt einer wendelförmigen Steuer- oder Auswurffeder ist konzentrisch zur Längsachse ausgerichtet und wird ebenfalls vom eingeschobenen Injektionsgerät durchdrungen. Ein distales Ende des Federelements 23 stützt sich am Modulgehäuse 20 ab, ein proximales Ende des Federelements 23 drängt, über senkrecht zur Detektionsrichtung ausgebildete Halteflächen, das Steuerelement 26 und eine Ausprägung 11b am Karpulenhalter 11 in Anschlag mit einem Halteelement auf der Innenseite des Arms 25b des Löseelements 25, wodurch das Injektionsgerät im Zusatzmodul axial festgeklemmt ist. Das Halteelement ist mit dem Löseelement einstückig ausgebildet oder zumindest in Richtung einer Verriegelungs-/Lösebewegung mit diesem starr gekoppelt Eine bügelförmige Erweiterung des Steuerelements 26 umfasst eine Querstrebe 26a, neben welcher ein Detektor 24 auf einer Leiterplatte 27 axialfest im Modulgehäuse 20 angeordnet ist.

Durch den Kraftschluss zwischen dem Steuerelement 26 und der Ausprägung 11b wird eine Bewegung des Injektionsgeräts 1 in distale Richtung gegenüber dem Modulgehäuse 20 auf das Steuerelement 26 übertragen. Unter leichter zusätzlicher Kompression des Federelements 23 wird das Steuerelement 26 gegenüber dem Modulgehäuse 20 nach distal verschoben, und der Detektor 24 durch die Querstrebe 26a ausgelöst. In dieser Ausführungsform ist das Steuerelement 26 Teil der zweiten Modulkomponente, während das Modulgehäuse 20 zur ersten Modulkomponente gehört.

In einem Lösezustand (nicht gezeigt) ist eine Ausprägung in Form eines Sperrnockens 26b des Steuerelements 26 in einer Ausnehmung 25c des Löseelements 25 eingerastet, so dass sich dieses trotz der Vorspannung durch die Rückstellfeder gegen eine Bewegung nach oben gesperrt ist. Sobald das Injektionsgerät aus Richtung proximal eingeführt wird und mit Ausprägungen 11b das Steuerelement 26 unter initialer Kompression des Federelements 23 nach distal schiebt, wird der Sperrnocken 26b axial aus der Ausnehmung 25c geschoben und das Löseelement 25 entsperrt. Das Löseelement 25 wird durch die Rückstellfeder nach oben gedrängt und hintergreift mit seinen Halteelementen die seitlichen Ausprägungen 11b am Injektionsgerät. Dadurch wird das Zusatzmodul gegenüber dem Injektionsgerät im Haltezustand verriegelt. Zur Freigabe des Zusatzmoduls muss das Löseelement 25 durch Druck auf die Druckflächen zur Längsachse hin nach unten gedrückt werden, bis die Verrieglung durch die Halteelemente freigegeben ist beziehungsweise Freigabeelemente oder Ausnehmungen in den Armen 25b mit den Ausprägungen 11b ausgerichtet sind. Die Sperrnocken 26b rasten wieder ein, das Federelement 23 entspannt sich und schiebt das Injektionsgerät aus dem Zusatzmodul. Alternativ kann der Sperrnocken auch am Löseelement vorgesehen sein und in eine Ausnehmung des Steuerelementes eingreifen.

Fig.4 zeigt eine Schrägaufsicht eines Teils eines Injektionsgeräts 1 mit Gerätegehäuse 10 und Karpulenhalter 11 sowie ein auf das Injektionsgerät aufgesetztes und mit diesem in einem Haltezustand verriegeltes Zusatzmodul 2. Eine vordere Hälfte des Modulgehäuses 20 wurde entfernt und gibt den Blick frei auf ein Löseelement 25 mit einer nach oben gerichteten Bedienfläche 25a. Im Unterschied zu Fig.3 sind in dieser Ausführungsform die Rückstellfeder und die Steuerfeder in einer einzigen Feder kombiniert und das Steuerelement drehbar statt axialverschiebbar gelagert. Das Steuerelement 26 verfügt über eine Drehachse 26c, mit welcher es im Modulgehäuse 20 drehbar verankert ist, und um welche es von der Löseposition in die gezeigte Halteposition verkippt werden kann wenn das Injektionsgerät nach distal geschoben wird relativ zum Modulgehäuse. Die vom Steuerelement 26 gebildete Öffnung für das Injektionsgerät ist dazu in einer Richtung senkrecht zur Drehachse 26c leicht oval ausgebildet. Das Löseelement 25 umfasst wiederum zwei seitliche parallele Arme mit Ausnehmungen, in welche Sperrnocken des Steuerelements in der Löseposition eingreifen, und mit Halteelementen welche im Haltezustand Ausprägungen an den Armen des Löseelements hintergreifen (nicht dargestellt). Ein Federelement 23 in Form einer Schenkelfeder drückt mit einem ersten Schenkel 23a den Druckknopf des Löseelements 25 gegen oben und mit einem zweiten Schenkel 23b das Steuerelements 26 an einem ausreichend von der Drehachse 24c entfernten Angriffspunkt gegen proximal. In der Löseposition ist die Bedienfläche 25a des Löseelements versenkt und die Schenkelfeder 23 gespannt. Im Haltezustand ist das Steuerelement fast senkrecht zur Längsachse ausgerichtet, die Druckfläche in einer Halteposition eben mit der Oberfläche des Modulgehäuses, und die Schenkelfeder 26 leicht entspannt, da eine Spannbewegung des ersten Schenkels 26a durch eine Entspannung des zweiten Schenkels 26b übertroffen wird. Auch in dieser Ausführungsform wird durch Drücken des Druckknopfs die Verriegelung der Ausprägungen 11c gelöst, das Injektionsgerät nach proximal geschoben und der Knopf in eingedrückter Löseposition gesperrt.

### BEZUGSZEICHENLISTE

| | | | |
|---|---|---|---|
| 1 | Injektionsgerät | 22 | Zweite Modulkomponente |
| 10 | Gerätegehäuse | 23 | Federelement |
| 10a | Auflagefläche | 24 | Detektor |
| 11 | Karpulenhalter | 25 | Löseelement |
| 11a | Nocken | 25a | Bedienfläche |
| 11b | Ausprägung | 25b | Arm |
| 12 | Dosiswahlknopf | 25c | Ausnehmung |
| 13 | Dosisanzeige | 26 | Steuerelement |
| 14 | Ausschüttknopf | 26a | Querstrebe |
| 15 | Gerätekappe | 26b | Sperrnocken |
| 2 | Zusatzmodul | 26c | Drehachse |
| 20 | Modulgehäuse | 27 | Leiterplatine |
| 21 | Erste Modulkomponente | | |

## Patentansprüche

1. Zusatzmodul (2) für ein Injektionsgerät (1) mit einer Längsachse und einem an einem proximalen Ende des Injektionsgeräts angeordneten und in distale Richtung entlang der Längsachse bewegbaren Ausschüttknopf (14) zur Ausschüttung eines flüssigen Medikaments aus einem Behälter des Injektionsgeräts, wobei das Zusatzmodul (2) eine Sensoreinheit zur Erkennung von Vorgängen oder Zuständen im Injektionsgerät umfasst und derart ausgebildet ist, dass der Ausschüttknopf (14) auch bei aufgesetztem Zusatzmodul berührt werden kann, **dadurch gekennzeichnet dass** das Zusatzmodul weiter umfasst
- eine erste Modulkomponente (21) und eine zweite Modulkomponente (22), welche durch eine Aktion des Anwenders und unter Spannung eines Federelements (23) in einer Bewegungsrichtung relativ zueinander bewegt werden können, und
- einen Detektor (24) zur Detektion dieser Relativbewegung, wobei
- die erste Modulkomponente (21) ein Modulgehäuse (20) des Zusatzmoduls mit einem Griff zum Ergreifen und Halten des Zusatzmoduls durch den Anwender umfasst, und die zweite Modulkomponente (22) in Bewegungsrichtung an ein Gerätegehäuse (10) des Injektionsgeräts (1) lösbar fixierbar ist, und wobei
- die Sensoreinheit nach Detektion einer Relativbewegung der Modulkomponenten von einem ersten in einen zweiten Betriebsmodus wechselt.

2. Zusatzmodul nach Anspruch 1, **dadurch gekennzeichnet, dass** der zweite Betriebsmodus der Sensoreinheit gegenüber dem ersten Betriebsmodus einen erhöhten Energieverbrauch, eine verbesserte Empfindlichkeit der Vorgangserkennung im Injektionsgerät, und/oder eine höhere Datenrate aufweist.

3. Zusatzmodul nach Anspruch 1 oder 2, **dadurch gekennzeichnet, dass** der Detektor (24) ausgebildet und/oder angeordnet ist zur Detektion einer Relativbewegung der Modulkomponenten (21, 22) parallel zu einer Längsrichtung des Injektionsgeräts (1).

4. Zusatzmodul nach einem der Ansprüche 1 bis 3, **dadurch gekennzeichnet, dass** die Sensoreinheit einen Sensor zur Erkennung von Bewegungen des Injektionsgeräts (1) und/oder von Vibrationen im Injektionsgerät umfasst.

5. Zusatzmodul nach einem der Ansprüche 1 bis 4, **dadurch gekennzeichnet, dass** das Zusatzmodul (2) ausgebildet ist zur Aufnahme des Injektionsgeräts (1) durch Einschieben des Injektionsgeräts in distale Richtung in den Aufnahmebereich, und dass das Zusatzmodul ein Löseelement (25) aufweist welches in einer Verriegelungsbewegung eine Haltefläche des Injektionsgeräts (1) hintergreift.

6. Zusatzmodul nach Anspruch 5, **dadurch gekennzeichnet, dass** die zweite Modulkomponente ein Steuerelement (26) umfasst, welches beim Einschub des Injektionsgeräts (1) in das Zusatzmodul (2) unter Spannung des Federelements (23) bewegt wird und die Verriegelungsbewegung des Löseelements (25) freigibt.

7. Zusatzmodul nach Anspruch 5, **dadurch gekennzeichnet, dass** das Federelement (23) die Verriegelungsbewegung des Löseelements (25) bewirkt.

8. Zusatzmodul nach einem der Ansprüche 1 bis 7, **dadurch gekennzeichnet, dass** die erste oder die zweite Modulkomponente (21, 22) durch zwei gleich starke Federelemente in einer Mitteposition relativ zur anderen der ersten oder zweiten Modulkomponente (21, 22) gehalten ist, so dass die zwei Modulkomponenten (21, 22) in zwei entgegengesetzte Detektionsrichtungen relativ zueinander bewegbar sind.

9. Zusatzmodul nach einem der Ansprüche 1 bis 8, **dadurch gekennzeichnet, dass** die Relativbewegung zwischen der ersten und der zweiten Modulkomponente einen Hub aufweist, welcher weniger als 3 mm beträgt, bevorzugt weniger als 1 mm, in axialer Richtung oder weniger als 15° beträgt, bevorzugt weniger als 5°, in Drehrichtung.

10. Injektionssystem umfassend ein Injektionsgerät (1) mit einem an einem proximalen Ende des Injektionsgeräts angeordneten Ausschüttknopf (14) zur Ausschüttung eines flüssigen Medikaments aus einem Behältnis des Injektionsgeräts, und umfassend ein Zusatzmodul (2) nach einem der Ansprüche 1 bis 9, wobei die zweite Modulkomponente (22) des Zusatzmodul (2) an einem Gerätegehäuse (10) des Injektionsgeräts (1) lösbar fixiert ist.

11. Injektionssystem nach Anspruch 10, **dadurch gekennzeichnet, dass** die zweite Modulkomponente (22) mittels Kraftschluss flächig auf das Gerätegehäuse (10) gepresst wird.

12. Injektionssystem nach Anspruch 10, **dadurch gekennzeichnet, dass** die zweite Modulkomponente (22) mittels Formschluss an Aus- oder Einprägungen des Injektionsgeräts (1) angebracht ist.

13. Injektionsgerät zur Ausschüttung eines flüssigen Medikaments aus einem Behältnis des Injektionsgeräts, mit einer Sensoreinheit zur Erkennung von Vorgängen oder Zuständen im Injektionsgerät, einer ersten Gerätekomponente umfassend einen Griff zum Ergreifen und Halten des Injektionsgeräts durch den Anwender, einer zweiten Gerätekomponente, welche durch eine Aktion des Anwenders und unter Spannung eines Federelements in einer Bewegungsrichtung relativ zur ersten Gerätekomponente bewegt werden kann, und einen Detektor zur Detektion dieser Relativbewegung, wobei die Sensoreinheit nach Detektion einer Relativbewegung der Gerätekomponenten von einem ersten in einen zweiten Betriebsmodus wechselt.

## Claims

1. Add-on module (2) for an injection device (1), having a longitudinal axis and a dispensing button (14), which is arranged at a proximal end of the injection device and is movable in a distal direction along the longitudinal axis, for dispensing a liquid drug from a container of the injection device, wherein the add-on module (2) comprises a sensor unit for detecting processes or conditions in the injection device and is designed such that the dispensing button (14) can be touched even when the add-on module is attached, **characterized in that** the add-on module further comprises
- a first module component (21) and a second module component (22), which can be moved relative to each other in a direction of movement by means of an action of the user and while a spring element (23) is tensioned, and
- a detector (24) for detecting this relative movement, wherein
- the first module component (21) comprises a module housing (20) of the add-on module having a grip, by means of which the user grasps and holds the add-on module, and the second module component (22) is releasably fixable to a device housing (10) of the injection device (1) in the direction of movement, and wherein
- the sensor unit switches from a first to a second operating mode after a relative movement of the module components is detected.

2. Add-on module according to claim 1, **characterized in that,** compared to the first operating mode, the second operating mode of the sensor unit has higher energy consumption, improved sensitivity of process detection in the injection device, and/or a higher data rate.

3. Add-on module according to claim 1 or 2, **characterized in that** the detector (24) is designed and/or arranged for detecting a relative movement of the module components (21, 22) parallel to a longitudinal direction of the injection device (1).

4. Add-on module according to any of claims 1 to 3, **characterized in that** the sensor unit comprises a sensor for detecting movements of the injection device (1) and/or vibrations in the injection device.

5. Add-on module according to any of claims 1 to 4, **characterized in that** the add-on module (2) is designed to receive the injection device (1) by inserting the injection device into the receiving area in the distal direction, **and in that** the add-on module comprises a release element (25) which engages behind a holding surface of the injection device (1) in a locking movement.

6. Add-on module according to claim 5, **characterized in that** the second module component comprises a control element (26) which, when the injection device (1) is inserted into the add-on module (2), is moved while the spring element (23) is tensioned and enables the locking movement of the release element (25).

7. Add-on module according to claim 5, **characterized in that** the spring element (23) causes the locking movement of the release element (25).

8. Add-on module according to any of claims 1 to 7, **characterized in that** the first or the second module component (21, 22) is held in a central position, relative to the other of the first or second module component (21, 22), by means of two equally strong spring elements, such that the two module components (21, 22) are movable relative to each other in two opposite detection directions.

9. Add-on module according to any of claims 1 to 8, **characterized in that** the relative movement between the first and the second module component has a stroke of less than 3 mm, preferably less than 1 mm, in the axial direction or less than 15°, preferably less than 5°, in the direction of rotation.

10. Injection system comprising an injection device (1) having a dispensing button (14), which is arranged at a proximal end of the injection device, for dispensing a liquid drug from a container of the injection device, and comprising an add-on module (2) according to any of claims 1 to 9, wherein the second module component (22) of the add-on module (2) is releasably fixed to a device housing (10) of the injection device (1).

11. Injection system according to claim 10, **characterized in that** the second module component (22) is pressed flatly onto the device housing (10) by means of force fitting.

12. Injection system according to claim 10, **characterized in that** the second module component (22) is attached to protrusions or indentations of the injection device (1) by means of form fitting.

13. Injection device for dispensing a liquid drug from a container of the injection device, comprising a sensor unit for detecting processes or conditions in the injection device, a first device component comprising a grip, by means of which the user grasps and holds the injection device, a second device component which can be moved relative to the first device component in a direction of movement by means of an action of the user and while a spring element is tensioned, and a detector for detecting this relative movement, wherein the sensor unit switches from a first to a second operating mode after a relative movement of the device components is detected.

## Revendications

1. Module supplémentaire (2) pour un appareil d'injection (1) comportant un axe longitudinal et un bouton de distribution (14) disposé à une extrémité proximale de l'appareil d'injection et pouvant être déplacé dans la direction distale le long de l'axe longitudinal pour la distribution d'un médicament liquide à partir d'un récipient de l'appareil d'injection, dans lequel le module supplémentaire (2) comprend une unité formant capteur pour la reconnaissance de processus ou d'états dans l'appareil d'injection et est conçu de telle sorte que le bouton de distribution (14) peut être touché même lorsque le module supplémentaire est en place, **caractérisé en ce que** le module supplémentaire comprend en outre
- un premier composant de module (21) et un second composant de module (22) qui peuvent être déplacés l'un par rapport à l'autre dans une direction de déplacement par une action de l'utilisateur et par la tension d'un élément formant ressort (23), et
- un détecteur (24) pour la détection dudit mouvement relatif, dans lequel
- le premier composant de module (21) comprend un boîtier de module (20) du module supplémentaire comportant une poignée pour la saisie et le maintien du module supplémentaire par l'utilisateur, et le second composant de module (22) peut être fixé de manière amovible dans la direction de déplacement à un boîtier d'appareil (10) de l'appareil d'injection (1), et dans lequel
- l'unité formant capteur passe d'un premier mode de fonctionnement à un second mode de fonctionnement après la détection d'un mouvement relatif des composants de module.

2. Module supplémentaire selon la revendication 1, **caractérisé en ce que** le second mode de fonctionnement de l'unité formant capteur présente, par rapport au premier mode de fonctionnement, une consommation d'énergie accrue, une sensibilité améliorée de la reconnaissance de processus dans l'appareil d'injection, et/ou un débit de données plus élevé.

3. Module supplémentaire selon la revendication 1 ou 2,
**caractérisé en ce que** le détecteur (24) est conçu et/ou disposé pour la détection d'un mouvement relatif des composants de module (21, 22) parallèlement à une direction longitudinale de l'appareil d'injection (1).

4. Module supplémentaire selon l'une des revendications 1 à 3,
**caractérisé en ce que** l'unité formant capteur comprend un capteur pour la reconnaissance de mouvements de l'appareil d'injection (1) et/ou de vibrations dans l'appareil d'injection.

5. Module supplémentaire selon l'une des revendications 1 à 4,
**caractérisé en ce que** le module supplémentaire (2) est conçu pour recevoir l'appareil d'injection (1) par insertion de l'appareil d'injection dans la direction distale dans la zone de réception, et **en ce que** le module supplémentaire présente un élément de détachement (25) qui vient en prise par l'arrière avec une surface de retenue de l'appareil d'injection (1) dans un mouvement de verrouillage.

6. Module supplémentaire selon la revendication 5, **caractérisé en ce que** le second composant de module comprend un élément de commande (26) qui, lors de l'insertion de l'appareil d'injection (1) dans le module supplémentaire (2), est déplacé par la tension de l'élément formant ressort (23) et libère le mouvement de verrouillage de l'élément de détachement (25).

7. Module supplémentaire selon la revendication 5, **caractérisé en ce que** l'élément formant ressort (23) provoque le mouvement de verrouillage de l'élément de détachement (25).

8. Module supplémentaire selon l'une des revendications 1 à 7,
**caractérisé en ce que** le premier ou le second composant de module (21, 22) est maintenu dans une position centrale par rapport à l'autre parmi le premier ou le second composant de module (21, 22) par deux éléments formant ressorts de force égale, de sorte que les deux composants de module (21, 22) peuvent être déplacés l'un par rapport à l'autre dans deux directions de détection opposées.

9. Module supplémentaire selon l'une des revendications 1 à 8,
**caractérisé en ce que** le mouvement relatif entre le premier et le second composant de module présente une course qui est inférieure à 3 mm, de préférence inférieure à 1 mm, dans la direction axiale, ou qui est inférieure à 15°, de préférence inférieure à 5°, dans la direction de rotation.

10. Système d'injection comprenant un appareil d'injection (1) comportant un bouton de distribution (14) disposé à une extrémité proximale de l'appareil d'injection pour la distribution d'un médicament liquide à partir d'un récipient de l'appareil d'injection, et comprenant un module supplémentaire (2) selon l'une des revendications 1 à 9, dans lequel le second composant de module (22) du module supplémentaire (2) est fixé de manière amovible à un boîtier d'appareil (10) de l'appareil d'injection (1).

11. Système d'injection selon la revendication 10, **caractérisé en ce que** le second composant de module (22) est pressé à plat sur le boîtier d'appareil (10) au moyen d'une liaison à force.

12. Système d'injection selon la revendication 10, **caractérisé en ce que** le second composant de module (22) est monté au moyen d'une liaison par complémentarité de forme sur des évidements ou des creux de l'appareil d'injection (1).

13. Appareil d'injection pour la distribution d'un médicament liquide à partir d'un récipient de l'appareil d'injection, comportant une unité formant capteur pour la reconnaissance de processus ou d'états dans l'appareil d'injection, un premier composant d'appareil comprenant une poignée pour la saisie et le maintien de l'appareil d'injection par l'utilisateur, un second composant d'appareil qui peut être déplacé par une action de l'utilisateur et par la tension d'un élément formant ressort dans une direction de mouvement par rapport au premier composant d'appareil, et un détecteur pour la détection dudit mouvement relatif, dans lequel l'unité formant capteur passe d'un premier mode de fonctionnement à un second mode de fonctionnement après la détection d'un mouvement relatif des composants d'appareil.
